# EUROPÄISCHE PATENTANMELDUNG

(11) **EP 3 178 825 A1**
(43) Veröffentlichungstag der Anmeldung: **14.06.2017**
(21) Anmeldenummer: 15198143.8
(22) Anmeldetag: 07.12.2015
(51) Int. Cl.: C07F 9/6574

(54) **HETEROZYKLISCHE SELENA-PHOSPHITE SOWIE VERFAHREN ZU DEREN HERSTELLUNG**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); SELENT, Detlef, 18059 Rostock (DE); BÖRNER, Armin, 18059 Rostock (DE); WEILBEER, Claudia, AB T6C 4C8 Edmonton (CA)

(57) **Zusammenfassung**

Neue heterozyklische Selena-Phosphite, Verfahren zu deren Herstellung als auch deren Verwendung als Ligandenbaustein zur Herstellung von Liganden für eine Anwendung in Komplexen.

## Beschreibung

Neue heterozyklische Selena-Phosphite, Verfahren zu deren Herstellung als auch deren Verwendung als Ligandenbaustein zur Herstellung von Liganden für eine Anwendung in Komplexen.

Die Herstellung von an der Hydroxylgruppe ungeschützten Selenodiphenolen mit geringen Ausbeuten ist bekannt aus T. K. Paine et al., "Manganese complexes of mixed O, X, O-donor ligands (X = S or Se): synthesis, characterization and catalytic reactivity", Dalton Trans., 2003, 15, 3136-3144. T. K. Paine et al. beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von nur 25 % erhalten.

Eine weitere mehrstufige Syntheseroute unter Verwendung von Grignard-Reagenz offenbart H. M. Lin et al., "A novel and efficient synthesis of selenides", ARKIVOC, 2012, viii, 146-156. Es wird eine Syntheseroute für Selenobiarylether offenbart in der zunächst Brom an das entsprechende Phenol addiert werden muss, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt:

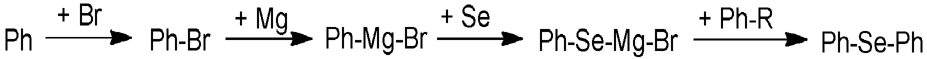

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Massstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Eine grosstechnisch wirtschaftliche Syntheseroute zur Herstellung von Selenodiphenolen beschreibt die EP 15168645.8 bzw. US 14/720,063.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxidierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et al., Journal of Catalysis, 2013, 298, 198-205). Diese zeichnen sich durch eine gute Aktivität aus, jedoch ist die n/i-Selektivität für endständig oxidierte Verbindungen gering und verbesserungswürdig.

In diesen Hydroformylierungen werden in der Regel Mono- und Bisphosphite eingesetzt, die oftmals aus Biphenol-Bausteinen aufgebaut sind. Die Entwicklung neuer Liganden ist häufig durch die zur Verfügung stehenden Biphenol-, also Ligandenbausteine limitiert. So stellen 2,2'-Selenobiarylether sowie Diphenylselenoxide und Diphenylselenide eine hochinteressante Verbindungsklasse dar. Die 2,2'-Selenobiarylether werden derzeit nur in bestimmten Komplexen, vor allem manganhaltigen, verwendet, sie besitzen aber ein großes Potential für weitere Anwendungen.

Aufgabe der Erfindung war es, eine weitere gänzlich neue Substanzklasse an Liganden und Ligandenbausteinen herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren herzustellen. Daher bestand auch die Aufgabe neue Zwischenprodukte als Ligandenbausteine zur Herstellung von Liganden bereitzustellen.

Gelöst werden die Aufgaben mit den heterozyklischen Selena-Phosphiten nach Anspruch 1, dem Verfahren nach Anspruch 6 sowie der Verwendung nach Anspruch 12. Besondere Ausführungsformen sind in den Unteransprüchen sowie detailliert in der Beschreibung offenbart. Gelöst werden die Aufgaben vorzugsweise mit Selena-Phosphiten der Strukturen **I**, **Ia, Ib, Ic** und **Id.** Dabei sind die Hydroxy- oder Chlor-funktionellen Selena-Phosphite der Struktur **I**, **Ia**, **Ib**, **Ic** und **Id** besonders bevorzugte Zwischenprodukte als Ligandenbaustein zur Herstellung von Liganden, wie beispielsweise von Phosphit-Liganden.

Gegenstand der Erfindung ist mindestens eine Verbindung eines heterozyklischen Selena-Phosphites der allgemeinen Struktur **I** 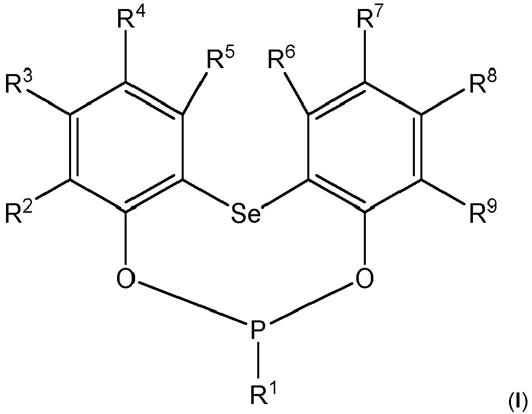 wobei R², R³, R⁴, R⁵, R⁶, R⁷, R⁸ und R⁹ jeweils unabhängig voneinander ausgewählt sind aus: -H,-(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte-(C₆-C₂₀)-Arylgruppe, insbesondere umfassend die Alkyl- und/oder Arylgruppen in -(C₁-C₁₂)-Alkyl, - O-(C₁-C₁₂)-Alkyl, -(C₆-C₂₀)-Aryl, -O-(C₆-C₂₀)-Aryl, -OC=O-(C₁-C₁₂)-Alkyl, mindestens einen Substituenten aufweisen kann und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -OH und -Hal, und wobei -Hal ausgewählt ist aus Fluor, Chlor, Brom, Jod, insbesondere bevorzugt sind Chlor und Brom, besonders bevorzugt Chlor, wobei optional die Verbindung **I** im Gemisch mit einem Umlagerungsprodukt der Struktur **I** vorliegt. Gleichfalls Gegenstand der Erfindung ist eine Zusammensetzung umfassend mindestens eine Verbindung der Struktur **I**, insbesondere umfassend eine Verbindung der Struktur **Ia** optional im Gemisch mit dem Umlagerungsprodukt gemäss der Struktur **Ia*** oder eine Verbindung der Struktur **Ib**.

Entsprechend einer Ausführungsvariante kann die Verbindung des heterozyklischen Selena-Phosphits der allgemeinen Struktur **I** als Verbindung der Struktur **Ia** im Gemisch mit einer Verbindung der Struktur **Ia*** vorliegen, 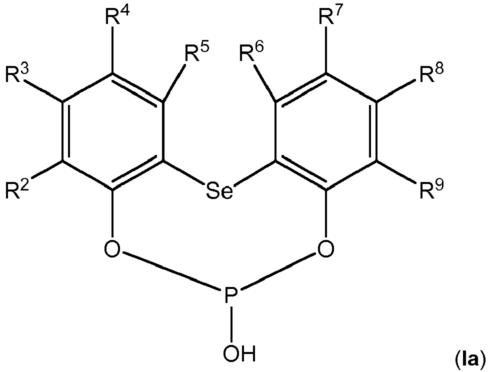 wobei R², R³, R⁴, R⁵, R⁶, R⁷, R⁸ und R⁹ in Struktur **Ia** jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN,-N[(C₁-C₁₂)-Alkyl]₂, wobei die Alkyl- und Arylgruppen jeweils unabhängig unsubstituiert oder substituiert kein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, 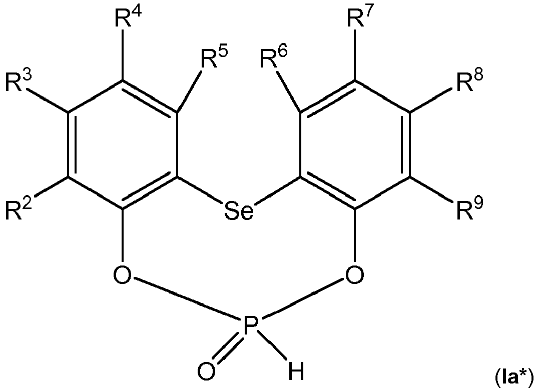 und wobei R², R³, R⁴, R⁵, R⁶, R⁷, R⁸ und R⁹ in Struktur **Ia*** jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Nach einer weiteren besonders bevorzugten Alternative kann das heterozyklische Selena-Phosphit der allgemeinen Struktur **I** als Verbindung der Struktur **Ib** vorliegen, 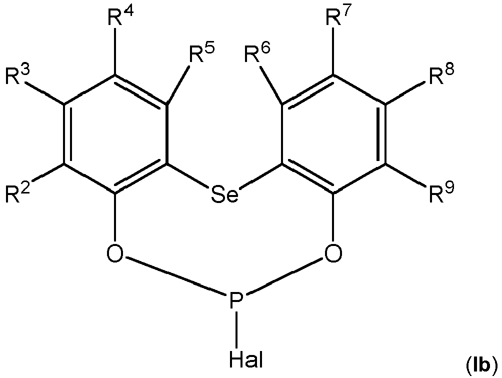 wobei R², R³, R⁴, R⁵, R⁶, R⁷, R⁸ und R⁹ in Struktur **Ib** jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und, wobei -Hal unabhängig ausgewählt ist aus Fluor, Chlor, Brom, Jod, insbesondere bevorzugt Chlor und Brom, besonders bevorzugt Chlor.

Des Weiteren kann es bevorzugt sein, wenn das heterozyklische Selena-Phosphit der allgemeinen Struktur **I** ausgewählt ist aus mindestens einer Verbindung der Struktur **Ic** und **Ic***, wobei optional die Verbindungen der Strukturen **Ic** und **Ic*** als Gemisch vorliegen können, 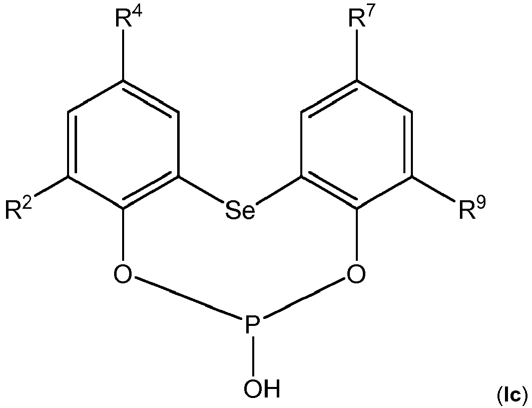 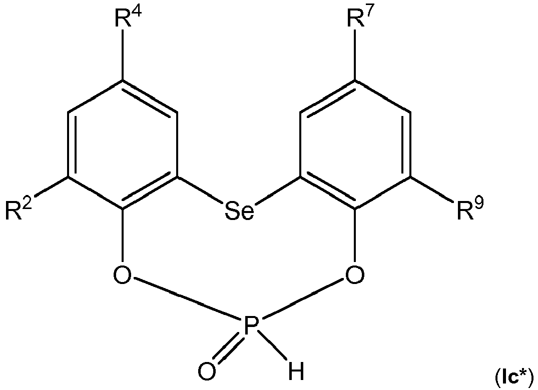 wobei R², R⁴, R⁷ und R⁹ in den Strukturen **Ic** und **Ic*** jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Vorzugsweise können R², R⁴, R⁷ und R⁹ in den Strukturen **Ic** und **Ic*** jeweils unabhängig voneinander ausgewählt sein aus: -(C₁-C₁₂)-Alkyl, -O-(C₁-C₁₂)-Alkyl, besonders bevorzugt sind R², R⁴, R⁷ und R⁹ Methyl. Alternativ können R², R⁴, R⁷ und R⁹ ausgewählt sein aus Methyl-, Ethyl, Propyl, *tert*-Butyl-, Methoxy-, und iso-Pentyl.

Entsprechend einer weiteren Alternative kann das heterozyklische Selena-Phosphit der allgemeinen Struktur I ausgewählt sein aus mindestens einer Verbindung der Struktur **Id**, 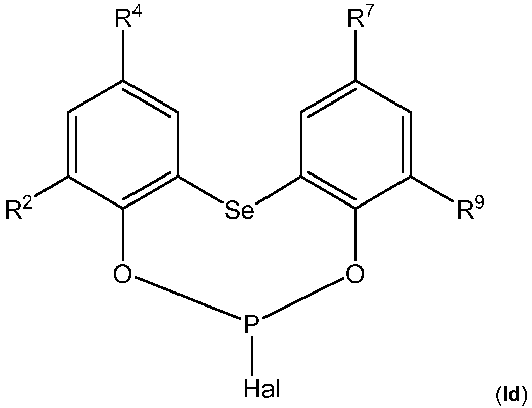 wobei R², R⁴, R⁷ und R⁹ in der Struktur **Id** jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, - COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl,-(C₃-C₁₂)-Heterocycloalkyl, -(C₆-C₂₀)-Aryl, Fluor, Chlor, Cyano, Formyl, Acyl oder Alkoxycarbonyl, wobei -Hal in Struktur **Id** ausgewählt ist aus Fluor, Chlor, Brom, Jod, vorzugsweise Chlor oder Brom, besonders bevorzugt Chlor. Vorzugsweise können R², R⁴, R⁷ und R⁹ in der Struktur **Id** jeweils unabhängig voneinander ausgewählt sein aus: -(C₁-C₁₂)-Alkyl, -O-(C₁-C₁₂)-Alkyl, besonders bevorzugt sind R², R⁴, R⁷ und R⁹ Methyl. Alternativ können R², R⁴, R⁷ und R⁹ ausgewählt sein aus Methyl-, Ethyl-, Propyl, *tert*-Butyl-, Methoxy-und iso-Pentyl.

Gleichfalls Gegenstand der Erfindung sind in den vorgenannten Strukturen der Selena-Phosphite und Selenodiarylen der Strukturen **I**, **Ia, Ia*** und **Ib** mit R², R³, R⁴, R⁵, R⁶, R⁷, R⁸ und R⁹, die jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert sind.

Gleichfalls Gegenstand der Erfindung sind in den vorgenannten Strukturen der Selena-Phosphite und Selenodiarylen der Strukturen **Ic**, **Ic*** und **Id** mit R², R⁴, R⁷ und R⁹, die jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert sind.

In einer Alternative sind R², R³, R⁴, R⁵, R⁶, R⁷, R⁸ und R⁹ in einem heterozyklischen Selena-Phosphit der allgemeinen Struktur **I**, **Ia**, **Ia*** und **Ib** jeweils unabhängig voneinander ausgewählt aus: -H und -(C₁-C₁₂)-Alkyl und/oder -O-(C₁-C₁₂)-Alkylgruppen, wobei die Alkylgruppen linear, verzweigt oder cyclisch sein können.

In einer Alternative sind R², R⁴, R⁷ und R⁹ in einem heterozyklischen Selena-Phosphit der allgemeinen Struktur **Ic**, **Ic*** und **Id** jeweils unabhängig voneinander ausgewählt aus: -H und -(C₁-C₁₂)-Alkyl und/oder -O-(C₁-C₁₂)-Alkylgruppen, wobei die Alkylgruppen linear, verzweigt oder cyclisch sein können.

Entsprechend einer besonders bevorzugten Ausführungsvariante sind R², R³, R⁴, R⁵, R⁶, R⁷, R⁸, R⁹ in den Selena-Phosphiten der Strukturen **I**, **Ia, Ia*** sowie **Ib** jeweils unabhängig voneinander ausgewählt aus: -H, unsubstituierten -(C₁-C₁₂)-Alkyl und/oder unsubstituierten -O-(C₁-C₁₂)-Alkylgruppen, wobei die Alkylgruppen linear, verzweigt oder cyclisch sein können.

Entsprechend einer besonders bevorzugten Ausführungsvariante sind R², R⁴, R⁷, R⁹ in den Selena-Phosphiten der Strukturen **Ic**, **Ic*** sowie **Id** jeweils unabhängig voneinander ausgewählt aus: -H, unsubstituierten -(C₁-C₁₂)-Alkyl und/oder unsubstituierten -O-(C₁-C₁₂)-Alkylgruppen, wobei die Alkylgruppen linear, verzweigt oder cyclisch sein können.

Weitere bevorzugte Selena-Phosphite umfassen Strukturen **I**:
(i) mit R², R³, R⁴, R⁵, R⁶, R⁷, R⁸, R⁹ die jeweils unabhängig voneinander ausgewählt sind aus: -H, -(C₁-C₁₂)-Alkyl, -O-(C₁-C₁₂)-Alkyl,
(ii) mit R², R³, R⁴, R⁵, R⁶, R⁷, R⁸, R⁹ die jeweils unabhängig voneinander ausgewählt sind aus: -H und -(C₁-C₁₂)-Alkyl oder -H und -O-(C₁-C₁₂)-Alkyl,
(iii) mit R², R⁴, R⁷, R⁹ die jeweils unabhängig voneinander ausgewählt sind aus: -(C₁-C₁₂)-Alkyl, -O-(C₁-C₁₂)-Alkyl, insbesondere der Struktur **Ic**, **Ic*** oder **Id**, vorzugsweise mit jeweils mit Alkyl -(C₁-C₆)-Alkyl linear, verzweigt oder cyclisch,
(iv) mit R², R⁴, R⁷, R⁹ die jeweils Methyl-, Ethyl-, *tert*-Butyl-, iso-Pentyl, sind und R³, R⁵, R⁶, R⁸ sind jeweils -H, wobei in den Alternativen (i), (ii), (iii) und (iv) vorzugsweise R¹ jeweils unabhängig voneinander ausgewählt ist aus: -Cl und -Br, bevorzugt ist Chlor..

Gleichfalls Gegenstand der Erfindung ist ein Verfahren zur Herstellung eines heterozyklischen Selena-Phosphits der allgemeinen Struktur **I** sowie die heterozyklischen Selena-Phosphite und Gemische ausgewählt aus den Strukturen **I**, **Ia, Ia***, **Ib, Ic**, **Ic*** und **Id** erhältlich nach einem Verfahren der Erfindung, 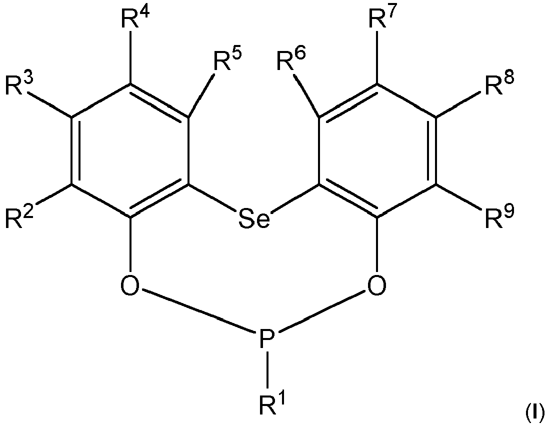 wobei R², R³, R⁴, R⁵, R⁶, R⁷, R⁸ und R⁹ in Struktur **I** jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und, insbesondere entsprechen die Reste R² bis R⁹ den Resten der Struktur **II**, wobei -R¹ unabhängig ausgewählt ist aus -OH und -Hal, wobei Hal ausgewählt ist aus Fluor, Chlor, Brom, Jod, insbesondere bevorzugt Chlor und Brom, besonders bevorzugt Chlor umfassend mindestens den Verfahrensschritt
(i) Umsetzen eines Selenodiaryls der allgemeinen Struktur **II** 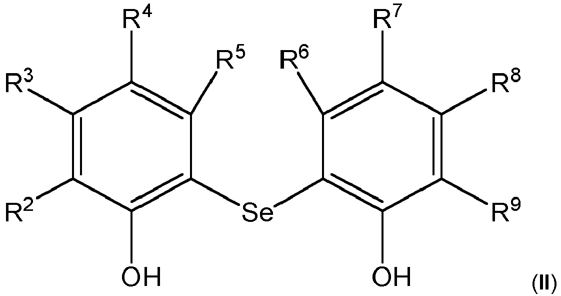 wobei R², R³, R⁴, R⁵, R⁶, R⁷, R⁸ und R⁹ in Struktur **II** jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit P(Hal)₃ der Formel **III**, wobei -Hal ausgewählt ist aus Fluor, Chlor, Brom, Jod, insbesondere bevorzugt Chlor und Brom, besonders bevorzugt Chlor, vorzugsweise erfolgt eine Umsetzung mit P(Hal)₃ der Formel **III** umfassend PCl₃ oder PBr₃, besonders bevorzugt PCl₃
(iii) und erhalten mindestens eines heterozyklischen Selena-Phosphits der allgemeinen Struktur **I** optional im Gemisch mit einem Umlagerungsprodukt des Selena-Phosphites der allgemeinen Struktur **I**.

Ferner ist Gegenstand der Erfindung ein Verfahren in dem das heterozyklische Selena-Phosphit der allgemeinen Struktur **I** als Verbindung der Struktur **Ia** wie vorstehend dargestellt im Gemisch mit einer Verbindung der Struktur **Ia*** erhalten wird.

Die Umsetzung im Verfahren erfolgt vorzugsweise in Gegenwart einer Base, insbesondere eines Amins oder einer Pyridinbase, insbesondere eines Alkylamins, wie Triethylamin oder Dimethylaminobutan, insbesondere Triethylamin

Des Weiteren erfolgt die Umsetzung vorzugsweise indem das Selenodiaryl der allgemeinen Struktur **II**, wie vorstehend offenbart, mit P(Hal)₃ der Formel **III** im molaren Verhältnis von 10 : 1 bis 1 : 10 umgesetzt wird, vorzugsweise im Verhältnis von 1,2 :1 bis 1: 1,2. Dabei ist es weiter bevorzugt, wenn die Umsetzung vorzugsweise im Temperaturbereich von -45 bis 80 °C durchgeführt, besonders von -15 bis 30°C, insbesondere von -5 bis 25°C.

Durch Wahl des im Verfahren eingesetzten Lösemittels kann die Bildung der Verfahrensprodukte in Richtung auf die Bildung des halogenierten heterozyklischen Selena-Phosphits der Struktur **Ib** oder **Id** oder alternativ in Richtung der Bildung der hydroxy-funktionellen heterozyklischen Selena-Phosphite **Ia** und **Ia***, **Ic** und **Ic*** gesteuert werden. Bei Verwendung von wasserfreien organischen aromatischen Kohlenwasserstoffen, wie Toluol, Xylol, etc. wird das halogenierte heterozyklische Selena-Phosphit erhalten, während mit aprotischen, wasserfreien Lösemitteln wie Ethern, THF überwiegend die hydroxy-funktionellen heterozyklischen Selena-Phosphite erhalten werden.

Nach einer Alternative ist Gegenstand der Erfindung ein Verfahren in dem die Umsetzung in einem aprotischen Lösemittel erfolgt, insbesondere ist das Lösemittel in einer Verfahrensalternative ausgewählt aus a) organischen aromatischen, halogenierten Lösemitteln oder Kohlenwasserstoffen und in einer zweiten Alternative b) aus Ethern, wie Diethylether, THF, Estern ,Ketonen.

Entsprechend einer weiteren Ausführungsform ist Gegenstand der Erfindung die Verwendung mindestens eines heterozyklischen Selena-Phosphits der vorgenannten Strukturen **I**, **Ia, Ia***, **Ib, Ic**, **Ic*** und **Id** oder der Zusammensetzungen erhältlich nach dem erfindungsgemäßen Verfahren zur Herstellung von Liganden, insbesondere als Ligandenbaustein zur Herstellung von Phosphitliganden. Besonders bevorzugt sind die Selena-Phosphite der Struktur **Ia, Ib, Ic** und **Id** Ligandenbausteine und somit Zwischenprodukte zur Herstellung von Liganden, wie Phosphit-Liganden.

Ein oder mehrere Substituenten in den vorgenannten Strukturen der Selena-Phosphite und Selenodiarylen umfassen vorzugsweise 1 bis 10 Substituenten, insbesondere 1 bis 3.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Halogen umfasst Fluor, Chlor, Brom und Jod, wobei Chlor und Fluor besonders bevorzugt sind.

Alle Erläuterungen zum Ausdruck -(C₁-C₁₂)-Alkyl in den vorgenannten erfindungsgemäßen Strukturen der Selena-Phosphite und Selenodiarylen gelten auch für die Alkylgruppen in -O-(C₁-C₁₂)-Alkyl, also in -(C₁-C₁₂)-Alkoxy.

Bevorzugt sind unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen in den vorgenannten Strukturen der Selena-Phosphite und Selenodiarylen können in Abhängigkeit von ihrer Kettenlänge, einen oder mehrere Substituenten aufweisen. Die Substituenten sind vorzugsweise unabhängig voneinander ausgewählt aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Alle Erläuterungen zum Ausdruck -(C₆-C₂₀)-Aryl in den vorgenannten erfindungsgemäßen Strukturen der Selena-Phosphite und Selenodiarylen gelten auch für die Arylgruppen in -O-(C₆-C₂₀)-Aryl.

Bevorzugt sind unsubstituierte -O-(C₆-C₂₀)-Gruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltigen Gruppen sind vorzugsweise ausgewählt aus -O-, -S-, -N-, -N(=O)-, -C(=O)- oder -S(=O)-. Beispiele für -(C₃-C₁₂)-Heterocycloalkylgruppen sind Tetrahydrothiophenyl, Tetrahydrofuryl, Tetrahydropyranyl und Dioxanyl.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter*, Art.-Nr. BIE 073107033) Ethylacetat (99.5%, Fa. *Walter*, Art.-Nr. BIE 003917025) und *n-*Hexan (95%, Fa. *Walter (Baker)*, Art.-Nr. 8669), *n*-Heptan (95%, Fa. *Walter (Baker)*, Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-ds (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co. KG* verwendet. Diese wurden, sofern nicht anders vermerkt, ohne weitere Reinigung eingesetzt.

Filtration: Filtrationen zur Abtrennung von entstandenen Feststoffen wurden mit einer G4-Fritte (Porenweite: 10-16 µm) durchgeführt.

### Analytik

IR-Spektroskopie: Die IR-Spektren wurden mit dem FT-IR-Spektrometer *Nicolet 6700* der Firma *Thermo Electron* aufgenommen. Die Substanzen wurden mittels ATR-Verfahren vermessen.

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.

¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm) wobei die chemischen Verschiebungen den ¹H-breitbandentkoppelten Spektren entnommen wurden.

⁷⁷Se-NMR-Spektroskopie: Die ⁷⁷Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.

Massenspektrometrie: EI-Massespektren wurden am Gerät *Finnigan MAT 95-XP* der Firma *Thermo Electron* sowie ESI-TOF-Massespektren mit dem Modell *6210 Time-of Flight LC*/*MS* der Firma *Agilent* aufgenommen.

### Röntgenkristallstrukturanalyse von der Verbindung der Struktur Id (2c)

Die Datensammlung erfolgte auf einem Bruker Kappa APEX II Duo Diffraktometer. Die Struktur wurde mit den direkten Methoden gelöst (SHELXS-97: G. M. Sheldrick, Acta Cryst. 2008, A64, 112-122.) und mit voller Matrix nach der Methode der kleinsten Fehlerquadrate gegen *F*² verfeinert (SHELXL-2014: G. M. Sheldrick, Acta Cryst. 2015, C71, 3-8.)

### Allgemeine Arbeitsvorschrift

8.2 mmol des jeweiligen Phenols werden im entsprechenden Lösungsmittel gelöst (8.2 m). Das Reaktionsgemisch wird erhitzt und 4.9 mmol Selendioxid werden unter Rühren zugegeben. Das Lösungsmittel wird im Vakuum destilliert (Temperatur <70° C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte waschen und in Fraktionen gesammelt. Die Fraktionen die das Produkt enthalten werden zusammengefasst und destillativ vom Laufmittel befreit. Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt Selenodiaryl (**II**) bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt der allgemeinen Struktur **II.**

### Bis(3,5-dimethyl-2-hydroxyphenyl)selen (II)

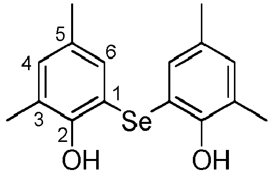

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.

¹H-NMR (400 MHz, CDCl₃): δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃); ¹³C-NMR (100 MHz, CDCl₃): δ (ppm) = 151.7 (C-2),133.2 (C-3), 133.1 (C-5), 130.4 (C-4), 124.2 (C-6), 114.9 (C-1), 20.3 (5-CH₃), 16.5 (3-CH₃); ⁷⁷Se-NMR (76 MHz, CDCl₃): δ (ppm) = 163.36 ppm.

### Bis(3-tert-butyl-5-methyl-2-hydroxyphenyl)selen (II)

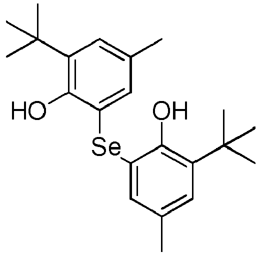

Die Durchführung der Reaktion erfolgt gemäß der allgemeinen Arbeitsvorschrift in einem verschraubbaren Reagenzglas. Dazu wurden 1.32 g (8.0 mmol, 1.0 Äquiv.) 2-*tert*-Butyl-4-methylphenol und 0.54 g (4.9 mmol, 0.6 Äquiv.) Selendioxid in 1 mL Pyridin gelöst und erhitzt.

¹H-NMR (300 MHz, CDCl₃): δ (ppm) = 7.15 (s, 2H, 6-H), 7.05 (s, 2H, 4-H), 5.07 (s,2H, OH), 2.21 (s, 6H, 5-CHs), 2.21 (s, 18H, 3-C(CH₃)_{3;} ¹³C-NMR (75 MHz, CDCl₃): δ(ppm))= 152.1, 136.4, 133.4, 120.1, 129.5, 117.2, 35.1, 29.6, 20.8.

### 3,3',5,5'-Tetra-tert-butylbiphenyl-2,2'-diol (II)

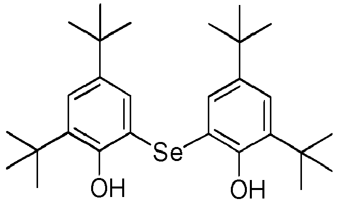

Die Durchführung der Reaktion erfolgt gemäß der allgemeinen Arbeitsvorschrift in einem verschraubbaren Reagenzglas. Dazu wurden 1.67 g (8.2 mmol, 1.0 Äquiv.) 2,4-Di-*tert*-butylphenol und 0.55 g (4.9 mmol, 0.6 Äquiv.) Selendioxid in 1 mL Pyridin gelöst und erhitzt.

¹H-NMR (400 MHz, CDCl₃): δ (ppm) = 7.31 (d, J=2.4 Hz, 2H), 7.29 (d, J=2.4), 6.29 (s, 2H), 1.42 (s, 18H), 1.24 (s, 18H); ¹³C-NMR (75 MHz, CDCl₃): δ (ppm) = 151.7, 143.5, 135.8, 129.8, 125.6, 117.2, 35.4, 34.4, 31.6, 29.7.

**Darstellung eines Selena-heterocyclischen Monophosphites der Struktur I**

### Synthese von 2,4,8,10-Tetramethyldibenzo[d,g][1,3,6,2]dioxaselenaphosphocin-6-ol Ic (2a) bzw. 2,4,8,10-Tetramethyldibenzo[d,g][1,3,6,2]dioxaselenaphosphocin-6-oxid Ic* (2b) bzw. 6-Chloro-2,4,8,10-tetramethyldibenzo[d,g][1,3,6,2]dioxaselenaphosphocin Id (2c)

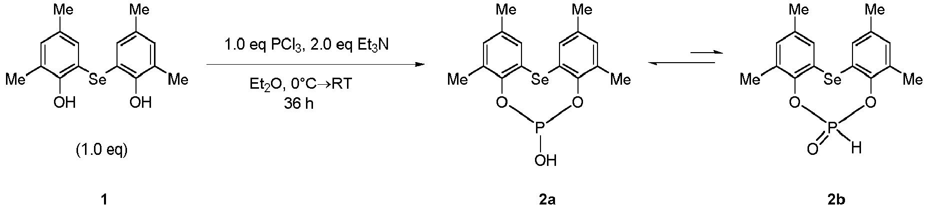

In einem ausgeheizten, unter Argon-Atmosphäre befindlichen 50 mL Schlenkgefäß wurden 87.5 µL (137 mg, 1.00 mmol, 1.0 eq) Phosphortrichlorid und 322 mg (1.00 mmol, 1.0 eq) Selenodiphenol 1 in 15 mL abs. Diethylether gelöst. Die hellgelbe Lösung wurde auf 0°C gekühlt und eine Lösung von 277 µL (202 mg, 2.00 mmol, 2.0 eq) Triethylamin in 2.5 mL abs. Diethylether zugetropft, wobei die Bildung eines farblosen Niederschlages beobachtet wurde. Es wurde mit 4.0 mL abs. Diethylether nachgespült und zehn Minuten bei 0°C gerührt. Die Reaktionslösung wurde anschließend auf RT erwärmt und weitere 36 Stunden gerührt. Der entstandene Niederschlag wurde abfiltriert und der Feststoff mit 5.0 mL abs. Diethylether gewaschen. Das Lösungsmittel wurde unter vermindertem Druck entfernt und das Rohprodukt drei Stunden im Vakuum bei 50°C getrocknet. Es wurden 386 mg (0.999 mmol, 99%) der Titelverbindungen **2a** und **2b** in einem Verhältnis von 85:15 (bestimmt aus ³¹P-NMR) als farbloser Feststoff erhalten.

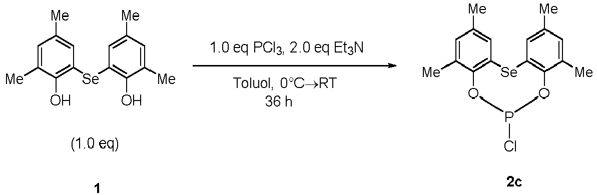

Wurde die Reaktion in abs. Toluol statt in abs. Diethylether (analoge Versuchsvorschrift) durchgeführt wurde 6-Chloro-2,4,8,10-tetramethyldibenzo[d,g][1,3,6,2]dioxaselena phosphocin **Id** (**2c**) erhalten. Die Verbindung ist kristallin und von den erhaltenen Kristallen der Verbindung **2c** konnte eine Einkristallstrukturanalyse angefertigt werden.

Gesamtes Reaktionsgemisch: **IR** (ATR): *v̂* (cm⁻¹) = 3205; 2916; 2853; 2730; 2465; 1460; 1423; 1376; 1272; 1191; 1115; 1037; 957; 934; 917; 887; 859; 812; 733; 671; 594; 579; 567; 526; 497; 475.

Gesamte Reaktionsgemisch: **⁷⁷Se**-**NMR** (57 MHz, Toluol-d₈): δ (ppm) = 313.5 ppm (d, *J*_{Se-P} = 59.7 Hz); 319.3 ppm (d, *J* = 4.38 Hz); 326.0 ppm (d, *J*_{Se-P} = 50.9 Hz).

Verbindungen **2a/2b**: **³¹P-NMR** (122 MHz, Toluol-d₈): δ (ppm) = 167.0 (*J*_{P-Se} = 59.5 Hz); -2.45.

Verbindungen **2a/2b: ¹H-gekoppeltes ³¹P-NMR** (122 MHz, Toluol-d₈): δ (ppm) = 167.0 (J_{P-Se} = 59.5 Hz); -2.41 (d, J_{P-H} = 745 Hz).

Gesamtes Reaktionsgemisch: **³¹P-NMR** (122 MHz, Toluol-d₈): δ (ppm) = 197.9 (d, J = 12.4 Hz); 167.0; 136.5; 136.0 (d, J = 12.4 Hz), -2.45.

Verbindungen **2a/2b: ESI-TOF/MS:** m/z = 369.016 ([M+H]⁺); 390.998 ([M+Na]⁺); 759.006 ([2M+Na]⁺).

Für Verbindungen **2a/2b: HR-MS (ESI-TOF):** ber. für C₁₆H₁₈O₃PSe ([M+H]⁺): 369.0154; gef.: 369.0157; ber. für C₁₆H₁₇O₃PSeNa ([M+Na]⁺): 390.99734; gef.: 390.99808.
Für Verbindungen **2a/2b: C₁₆H₁₇O₃PSe** (368.01 g/mol).
Für Verbindung **2c: C₁₆H₁₆ClO₂PSe** (385.97 g/mol).

Theoretische Berechnung zu den Verbindungen **2a/2b**:

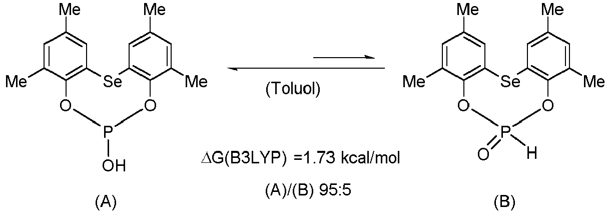

Kristallstrukturanalyse der Verbindung der Struktur **Id (2c):**

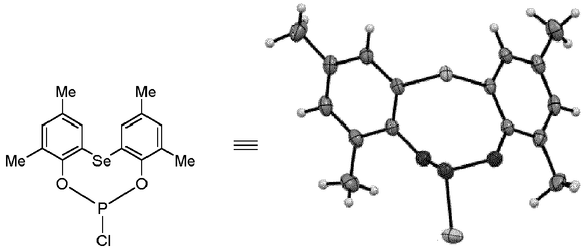

Verbindung **2c**: C₁₆H₁₆ClO₂PSe, *M* = 385.67, monoklin, Raumgruppe *P*2₁/*c*, *a* = 11.9587(5), *b* = 8.8151 (4), *c* = 15.6401 (7) Å, β = 93.4819(16)°, *V* = 1645.69(13) Å³, *Z* = 4, ρ_{ber}. = 1.557 g·cm⁻³, µ = 2.542 mm⁻¹, *T* = 150(2) K, 26607 gemessene, 3977 unabhängige Reflexe (*R*ᵢₙₜ = 0.0215), *R*₁ = 0.0245 (*l* > 2σ (*l*)), *wR*₂ = 0.0691 (alle Daten), 194 Parameter. Vermessbare Kristalle der Verbindung **2c** konnten in einem Lösungsmittel-Gemisch aus *n*-Heptan/Acetonitril (5:1) bei einer Temperatur von 6°C (Kühlschrank) erhalten werden.

## Patentansprüche

1. Verbindung eines heterozyklischen Selena-Phosphites, welches eine allgemeine Struktur (I) aufweist 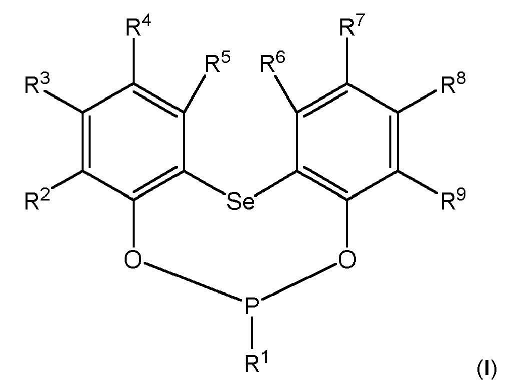 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl,-CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -OH und -Hal, und -Hal ausgewählt ist aus Fluor, Chlor, Brom, Jod, und, wobei optional die Verbindung der Struktur (**I**) im Gemisch mit einem Umlagerungsprodukt der Struktur (**I**) vorliegt.

2. Verbindung nach Anspruch 1, **dadurch gekennzeichnet, dass**
das heterozyklische Selena-Phosphit der allgemeinen Struktur (**I**) als Verbindung der Struktur (**Ia**) im Gemisch mit einer Verbindung der Struktur (**Ia***) vorliegt, 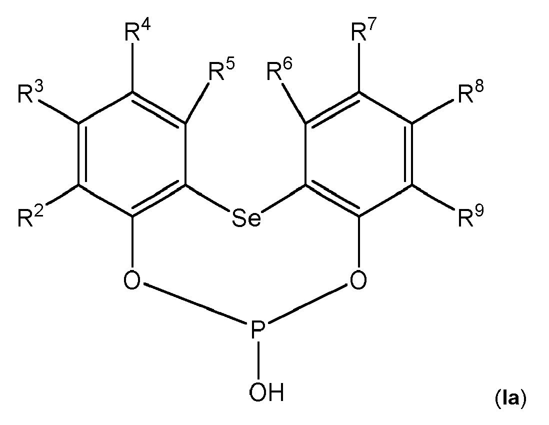 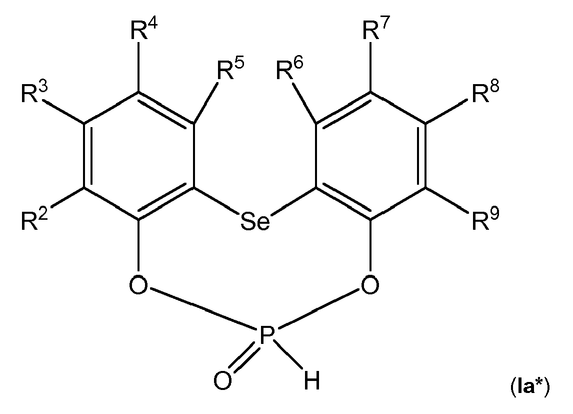 wobei R², R³, R⁴, R⁵, R⁶, R⁷, R⁸ und R⁹ in den Strukturen (**Ia**) und (**Ia***)jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl,-CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

3. Verbindung nach Anspruch 1, **dadurch gekennzeichnet, dass**
das heterozyklische Selena-Phosphit der allgemeinen Struktur (**I**) als Verbindung der Struktur (**Ib**) vorliegt, 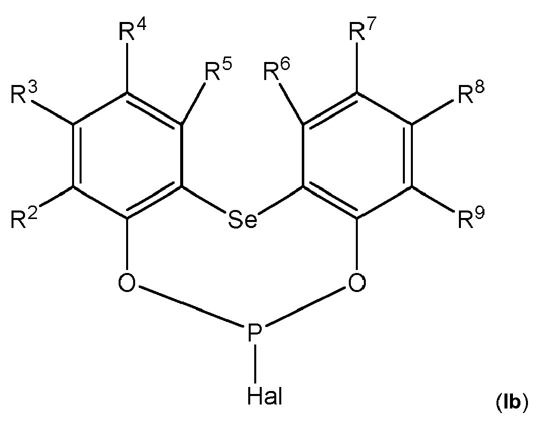 wobei R², R³, R⁴, R⁵, R⁶, R⁷, R⁸ und R⁹ in Struktur (**Ib**) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl,-Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, - COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -Hal ausgewählt ist aus Fluor, Chlor, Brom, Jod.

4. Verbindung nach Anspruch 1 oder 2, **dadurch gekennzeichnet, dass** das heterozyklische Selena-Phosphit der allgemeinen Struktur (I) ausgewählt ist aus mindestens einer Verbindung der Struktur (**Ic**), (**Ic***), wobei optional die Verbindungen der Strukturen (**Ic**) und (**Ic***) als Gemisch vorliegen, 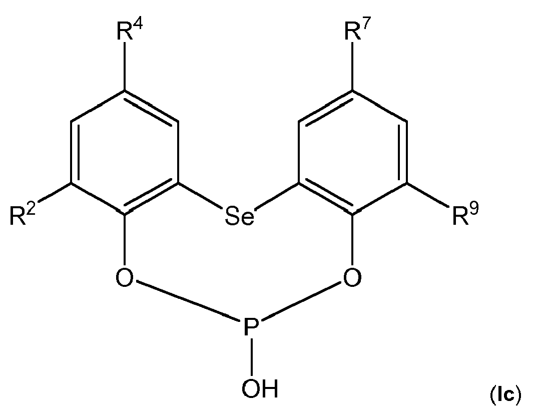 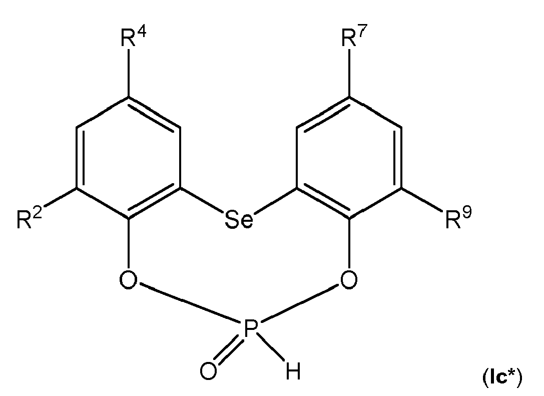 wobei R², R⁴, R⁷ und R⁹ in den Strukturen (**Ic**) und (**Ic***) jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl,-COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

5. Verbindung nach Anspruch 1 oder 2, **dadurch gekennzeichnet, dass** das heterozyklische Selena-Phosphit der allgemeinen Struktur (**I**) ausgewählt ist aus mindestens einer Verbindung der Struktur (**Id**), 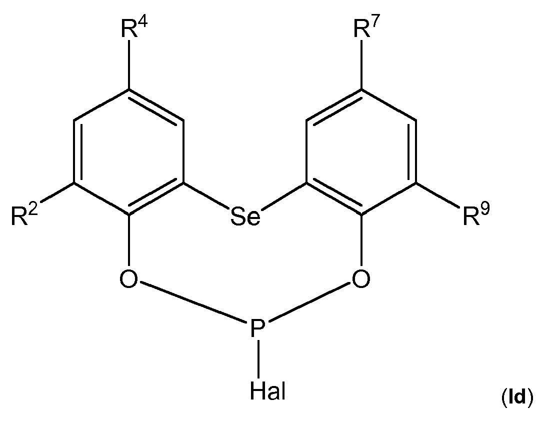 wobei R², R⁴, R⁷ und R⁹ in der Struktur (**Id**) jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, - SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, wobei -Hal ausgewählt ist aus Fluor, Chlor, Brom, Jod.

6. Verfahren zur Herstellung mindestens eines heterozyklischen Selena-Phosphit der allgemeinen Struktur (**I**) 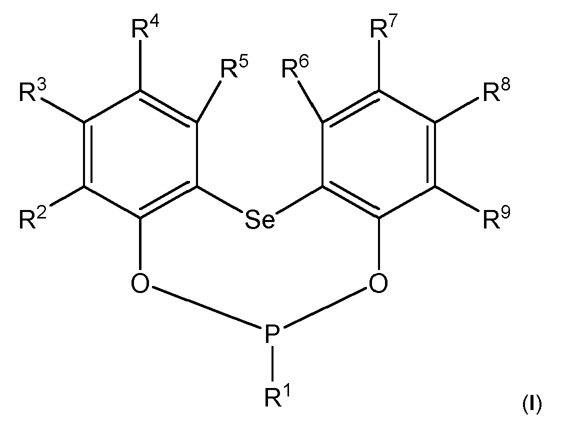 wobei R², R³, R⁴, R⁵, R⁶, R⁷, R⁸ und R⁹ jeweils unabhängig voneinander ausgewählt sind aus:-H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -OH und -Hal ist, wobei -Hal ausgewählt ist aus Fluor, Chlor, Brom, Jod, optional im Gemisch mit einem Umlagerungsprodukt der Verbindung der Struktur (**I**),
umfassend mindestens den Verfahrensschritt
(i) Umsetzen eines Selenodiaryls der allgemeinen Struktur (**II**) 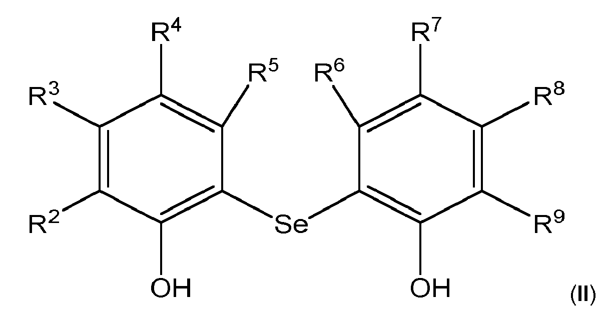 wobei R², R³, R⁴, R⁵, R⁶, R⁷, R⁸ und R⁹ in Struktur (**II**) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit P(Hal)₃ der Formel (**III**), wobei -Hal ausgewählt ist aus Fluor, Chlor, Brom, Jod,
(iii) und erhalten mindestens eines heterozyklischen Selena-Phosphits der allgemeinen Struktur (**I**) optional im Gemisch mit einem Umlagerungsprodukt der Verbindung der Struktur (**I**).

7. Verfahren nach Anspruch 6, **dadurch gekennzeichnet, dass**
das heterozyklische Selena-Phosphit der allgemeinen Struktur (**I**) als Verbindung der Struktur (**Ia**) im Gemisch mit einer Verbindung der Struktur (**Ia***) 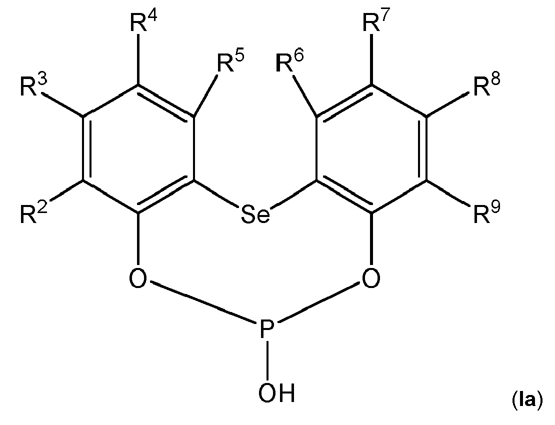 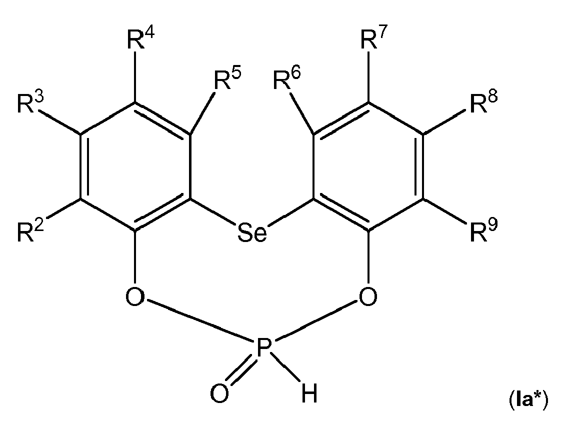 erhalten wird, wobei R², R³, R⁴, R⁵, R⁶, R⁷, R⁸ und R⁹ in Struktur (**Ia**) und (**Ia***) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

8. Verfahren nach Anspruch 6 oder 7,
wobei (i) die Umsetzung in Gegenwart eines Amins, insbesondere eines Alkylamins, wie Triethylamins erfolgt.

9. Verfahren nach einem der Ansprüche 6 bis 8, **dadurch gekennzeichnet,**
**dass** das Selenodiaryl der allgemeinen Struktur (**II**) nach Anspruch 6 mit P(Hal)₃ der Formel (**III**) im molaren Verhältnis von 10 : 1 bis 1 : 10 umgesetzt wird, vorzugsweise im Verhältnis von 1,2 :1 bis 1: 1,2.

10. Verfahren nach einem der Ansprüche 6 bis 9, **dadurch gekennzeichnet,**
**dass** P(Hal)₃ der Formel (**III**) PCl₃ oder PBr₃ ist.

11. Verfahren nach einem der Ansprüche 6 bis 10,
wobei (i) die Umsetzung zwischen -15 bis 30 °C erfolgt, insbesondere von -5 bis 5°C.

12. Verfahren nach einem der Ansprüche 6 bis 11,
wobei (i) die Umsetzung in einem aprotischen Lösemittel erfolgt, insbesondere ist das Lösemittel ausgewählt aus a) organischen aromatischen, halogenierten Lösemitteln oder Kohlenwasserstoffen, oder b) Ethern, THF, Estern oder Ketonen.

13. Verwendung eines heterozyklischen Selena-Phosphits nach einem der Ansprüche 1 bis 5 oder erhalten nach einem der Ansprüche 6 bis 12 zur Herstellung von Liganden, insbesondere als Ligandenbaustein zur Herstellung von Phosphitliganden.
